# EUROPEAN PATENT APPLICATION

(11) **EP 3 782 690 A1**
(43) Date of publication of application: **24.02.2021**
(21) Application number: 19192237.6
(22) Date of filing: 19.08.2019
(51) Int. Cl.: A61M 25/01

(54) **INTERNAL ARMATURE FOR A CATHETER**

(71) Applicant: VascoMed GmbH, 79589 Binzen (DE)
(72) Inventor: Bischoff, Robert, 79418 Schliengen (DE); Baureithel, Karl, 79664 Wehr (DE)
(74) Representative: Galander, Marcus

(57) **Abstract**

The disclosure relates to an internal armature (1) for a catheter (2) comprising a coil, at least one pull wire (20) for deflecting a tip section (4) of the catheter (2) and at least one connecting element (30) connecting the coil (10) to the pull wire (20), wherein at least a part of a distal end segment (23) of the pull wire is deflectable along a curve (C) when the pull wire (20) is moved along the longitudinal axis (L) in respect of the coil (10). Furthermore, the disclosure relates to a catheter (2) and a method of manufacture of the catheter (3).

## Description

The present disclosure relates to an internal armature for a catheter, a catheter and a method of manufacturing a catheter. In particular, the catheter may be adapted for electrophysiological therapy of cardiac rhythm disturbances.

In many applications of endovascular catheters, the tip of the catheter must be easily deflectable to adapt to the shape of the human vasculature. Additionally, in electrophysiological therapy of the heart, the catheter tip has to be precisely positioned within the heart for electrophysiological measurement and controlled ablation of heart tissue.

To this end, catheters of the prior art usually comprise a flexible tip section and a more rigid shaft section, which are manufactured from separate tubes and welded or fused together.

In addition, a spiral coil is sometimes placed within the catheter lumen at the transition between the shaft and the tip to provide stiffness to the sheath and transmit torque from the proximal end to the distal end of the catheter (document US 5,562,619).

To control the deflection of the catheter tip, one or several pull wires are commonly affixed to internal structures of the catheter tube or the head or ring electrode (if present). The physician may then manipulate the pull wires at the proximal end to achieve controlled deflection of the catheter tip.

Document US 5,273,535 describes a catheter where a pull wire is sandwiched between two leaf springs, providing additional stiffness to the pull wire.

The described catheters of the prior art have the disadvantage that their manufacturing process is relatively complicated, because several tube parts must be molded and additional tubes and/or stabilizing structures have to be inserted into the catheter interior. Furthermore, pull wires must be precisely fixed on internal components such as the catheter tip.

Therefore, the objective is to provide catheters with a deflectable tip which are improved in view of the described drawbacks of the prior art, in particular catheters which are easier to manufacture compared to catheters of the prior art.

This objective is obtained by the subject matter of the independent claims 1 (internal armature), 12 (catheter) and 15 (method of manufacture). Further embodiments are subject matter of dependent claims and are described hereafter.

A first aspect relates to an internal armature for a catheter comprising:
- a coil (e.g. a spiral coil) configured to be positioned in a lumen of a catheter, wherein the coil extends along a longitudinal axis from a proximal end to a distal end, and wherein the coil comprises an inner bore extending along the longitudinal axis, and
- at least one pull wire for deflecting or bending a tip section of the catheter, wherein the pull wire is at least partially positioned inside the inner bore of the coil, and wherein the pull wire comprises a proximal end segment configured to be connected, particularly fixed, to a manipulation device for moving the pull wire, particularly along the longitudinal axis, and a distal end segment protruding from the inner bore at the distal end of the coil.

The internal armature comprises at least one connecting element connecting, particularly fixing, the coil to the pull wire, wherein at least a part of the distal end segment of the pull wire is deflectable along a curve when the pull wire is moved axially along the longitudinal axis in respect of the coil, wherein particularly the at least one connecting element is fixed to the coil.

This has the advantage that the internal armature can be preassembled and installed together into a single tube having uniform flexibility along its length to form a catheter with a displaceable tip. By the invention, several steps of manufacture, such as providing several tubes, cutting the tubes and molding the tubes, can be omitted, and manufacture is therefore simplified.

In addition, the pull wire is already part of the internal armature and connected to the coil. Therefore, no separate fixing step of the pull wire to internal structures of the catheter is required, further simplifying manufacture of the catheter.

Since fewer components are needed to assemble a catheter, costs are optimized by the present invention.

Furthermore, by adjusting the arrangement between the pull wire, the connecting element and the coil, different curve forms of the catheter tip can be implemented, increasing adaptability of the catheter for different applications.

In the context of the present specification, the term "coil" describes a component comprising a wire like element, which is wound helically around a central longitudinal axis. The wire element may be tightly wound, such that the coil essentially resembles a tube without external application of force. Alternatively, gaps may be provided between neighboring windings of the coil. Furthermore, the wire coil may comprise one or several wire elements. For example, in case of one wire element, the coil may resemble a one start helix, and in case of two wire elements, the coil may resemble a two start helix. The coil provides stiffness to the shaft portion of the catheter, but may comprise a certain flexibility. For example, the shaft portion of the catheter comprising the coil will typically slightly bend when subjected to certain external force perpendicular to the longitudinal axis. The coil may be made of any suitable material.

The term "lumen of a catheter" as used herein refers to a hollow space within the catheter, such as the inside of the tube. A catheter may contain one lumen or several lumens. For instance, a typical catheter having more than lumen comprises an outer tube and at least one inner tube positioned in the outer tube. For example, the lumens of a catheter may be used to position tools, deliver fluids or lead electrical connects to electrodes at the tip.

In the context of the present specification, the term "pull wire" refers to an elongated member having a proximal end and a distal end and being adapted to deflect a catheter tip. Although the term "wire" is used herein, a pull wire according to the invention is not restricted to metallic material. In contrast, any suitable material, such as metal, plastic or fabric may be used for the pull wire.

The pull wire is deflectable along a curve. That means that in the absence of an external force, the pull wire extends essentially along a straight line, in particular parallel to the longitudinal axis. When an axial force is applied to the pull wire (i.e. from a manipulation device), the tip of the pull wire is moved perpendicular to the longitudinal axis, resulting in a deviation from a straight-line-shape at the distal tip of the pull wire.

Besides connecting, particularly fixing, the pull wire to the coil, the connecting element may provide or contribute to a restoring force exerted on the pull wire when the pull wire is deflected from the longitudinal axis, wherein the restoring force eventually results in the pull wire being moved back to a straight confirmation essentially parallel to the longitudinal axis.

According to an embodiment of the internal armature, the distal end segment of the pull wire is deflectable in a first lateral direction perpendicular to the longitudinal axis when the pull wire is moved, particularly pulled, along the longitudinal axis towards the proximal end of the coil, and wherein the distal end segment of the pull wire is deflectable in a second lateral direction perpendicular to the longitudinal axis and opposite the first lateral direction when the pull wire is moved, particularly pushed, along the longitudinal axis towards the distal end of the coil.

In other words, for example, the physician may move the distal end segment to the left by pulling on the pull wire and move the distal end segment to the right by pushing the pull wire. To which side the distal end segment is moved depends in particular on the arrangement between the pull wire, the connecting element and the coil.

In certain embodiments, the internal armature comprises a first pull wire and a second pull wire, wherein the first pull wire and the second pull wire are each connected, particularly fixed, to the coil by means of the at least one connecting element, and wherein the first pull wire and the second pull wire are each at least partially positioned inside the inner bore of the coil, and wherein the first pull wire and the second pull wire each comprise a respective proximal end segment configured to be connected, particularly fixed, to a manipulation device for moving the respective pull wire and a respective distal end segment protruding from the inner bore at the distal end of the coil, wherein the distal end segment of the first pull wire and the distal end segment of the second pull wire are jointly deflectable in a first lateral direction perpendicular to the longitudinal axis when the first pull wire is moved axially along the longitudinal axis in respect of the coil, and wherein the distal end segment of the first pull wire and the distal end segment of the second pull wire are jointly deflectable in a second lateral direction perpendicular to the longitudinal axis and opposite the first lateral direction when the second pull wire is moved axially along the longitudinal axis in respect of the coil.

A system with two pull wires improves control over deflection of the distal end segments and therefore of the catheter tip and facilitates manipulation by the physician.

According to some embodiments, the connecting element comprises a first end and a second end, wherein the first end of the connecting element is connected to the distal end of the coil, and wherein the second end of the connecting element is connected to the pull wire. In particular, the second end of the connecting element may be connected, particularly fixed, to a distal tip of the pull wire or, alternatively, the second end of the connecting element may be connected, particularly fixed, to an attachment point of the pull wire, wherein the pull wire extends beyond the attachment point.

In certain embodiments, the connecting element is connected, particularly fixed, to the at least one pull wire by means of a sleeve, wherein particularly the connecting element and/or the at least one pull wire is crimped, welded or soldered to the sleeve. Such a sleeve provides good fixation and the connection can be easily established also at small sizes. Alternatively, the connecting element can be melted together with the at least one pull wire, e.g. by laser melting. In this case, a sleeve is not necessary leading to a reduced size of the connecting element.

According to certain embodiments, the connecting element is a wire, particularly a metal wire, a wire strand or a braid. The braid may comprise seven wires. Braids may be bent reversibly and return to their original form when released.

In some embodiments, the connecting element is a spring element, particularly a leaf spring. This advantageously provides a restoring force to the pull wire, and thus to the catheter tip, after deflection.

In another embodiment, the internal armature comprises a positioning element, particularly a positioning ring, wherein a first section of the distal end segment of the pull wire between the distal end of the coil and the positioning element essentially extends along a straight line parallel to the longitudinal axis, and wherein a second section of the distal end segment of the pull wire between the positioning element and a distal tip of the pull wire is deflectable along a curve when the pull wire is moved axially along the longitudinal axis in respect of the coil.

This advantageously allows to implement many different curve shapes of the deflected distal end segment using the same internal armature by arranging the positioning element accordingly. For example, the positioning element can be placed at a desired position, and particularly fixed at this location, already during manufacturing of the catheter. Alternatively, the position of the positioning element can be adjusted during use of the catheter to generate different curve forms.

In certain embodiments, the positioning element is slidably connected to the pull wire. That is, the positioning element may be moved along the pull wire, particularly along the longitudinal axis.

In certain embodiments, the positioning element comprises at least one bore, wherein the pull wire and/or the connecting element can be placed in the bore, such that the pull wire and/or the connecting element extends through the bore. In another embodiment, the positioning element comprises at least one flexible tube, wherein the pull wire and/or the connecting element can be placed in the flexible tube, such that the pull wire and/or the connecting element extends through the flexible tube.

In certain embodiments, the internal armature comprises a positioning wire comprising a first end configured to be connected, particularly fixed, to a manipulation device for moving the positioning wire, particularly along the longitudinal axis, and a second end connected, particularly fixed, to the positioning element, such that the positioning element is movable relative to the pull wire by means of the positioning wire. A positioning wire is a means to easily and reliably adjust the position of the positioning element relative to the pull wire and thereby adjust the curve form of the catheter tip during operation of the catheter.

According to some embodiments, the internal armature comprises a bracing tube positioned around a partial segment of the distal end segment of the pull wire adjacent to the distal end of the coil in a circumferential direction in respect of the longitudinal axis, such that the partial segment is positioned essentially along a straight line parallel to the longitudinal axis by means of the bracing tube, wherein particularly the bracing tube comprises polyimide or is formed from polyimide. The bracing tube may comprise or be formed of polytetrafluoroethylene (PTFE), e.g. Teflon, fluorinated ethylene propylene (FEP), or polyether ether ketone (PEEK). The bracing tube provides stiffness to the catheter shaft and can therefore influence the resulting curve form of the catheter tip in an easy manner.

A second aspect relates to a catheter comprising an internal armature according to the first aspect and a lumen, wherein the coil of the internal armature is positioned in said lumen, and wherein the catheter comprises a tip section which is deflectable by deflecting at least a part of the distal end segment of the pull wire of the internal armature along a curve when the pull wire is moved axially along the longitudinal axis in respect of the coil of the internal armature.

In certain embodiments, the catheter further comprises a manipulation device connected, particularly fixed, to the proximal end segment of the pull wire of the internal armature, such that the pull wire is movable along the longitudinal axis by means of the manipulation device.

In certain embodiments, the tip section of the catheter comprises at least one electrode for providing an electric current. In particular, such electrodes can be used for electrophysiological measurements or electrophysiological treatment at the heart. In particular, the catheter may comprise a tip or head electrode positioned at the distal end of the catheter tip and at least one ring electrode positioned proximal to the head electrode at a distance from the head electrode. Typically, one or several ring electrodes are applied for electrophysiological measurements at the heart, particularly for locating diseased heart tissue, whereas the head electrode is used to ablate heart tissue. For electrophysiological measurements (diagnostics), a catheter may be provided with one or more ring electrodes in a distal region of the catheter (without a head electrode). Electrical leads providing a voltage to the electrodes from the proximal end of the catheter may be placed in specified internal lumens or tubes of the catheter.

A third aspect relates to a method of manufacturing a catheter, particularly according to the second aspect, wherein a single external tube is provided, and wherein an internal armature according to the first aspect is positioned within the external tube, wherein particularly the internal armature is connected, more particularly fixed, to the external tube.

Advantageously, the manufacturing process is greatly simplified by the internal armature according to the present disclosure because a single tube can be used to generate a catheter with a deflectable tip.

In certain embodiments of the method, at least one internal tube is subsequently positioned in the external tube. In this manner, multi-lumen catheters may be constructed. Particularly, the at least one internal tube comprises or consists of polyimide.

In the following section, preferred embodiments and further features are illustrated by means of the attached figures:
- Fig. 1: shows side views of a first embodiment of an internal armature;
- Fig. 2: shows side views of a second embodiment of an internal armature comprising a positioning element;
- Fig. 3: shows side and top views of further embodiments of an internal armature;
- Fig. 4: shows sectional views and top views of further embodiments of an internal armature with an additional lumen in the coil;
- Fig. 5: displays an embodiment of a catheter comprising an internal armature.

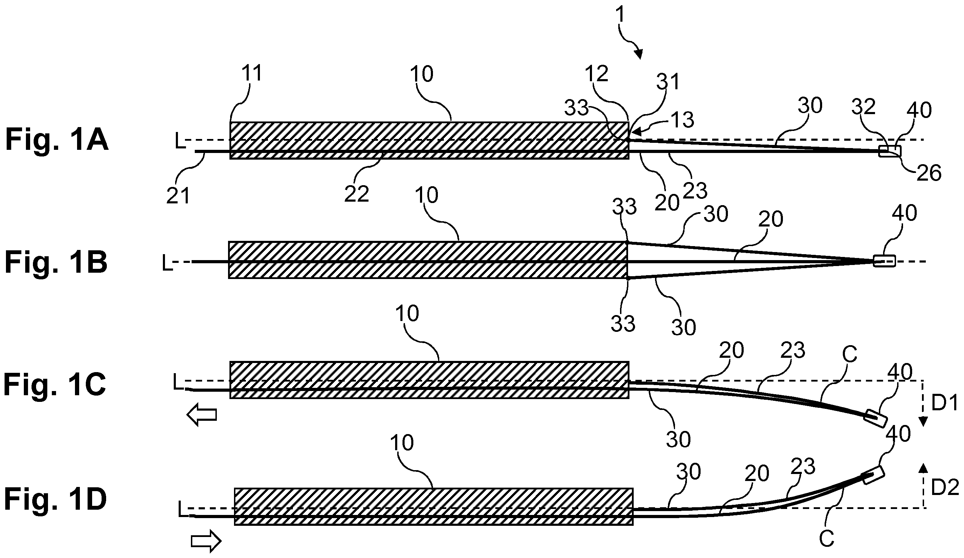

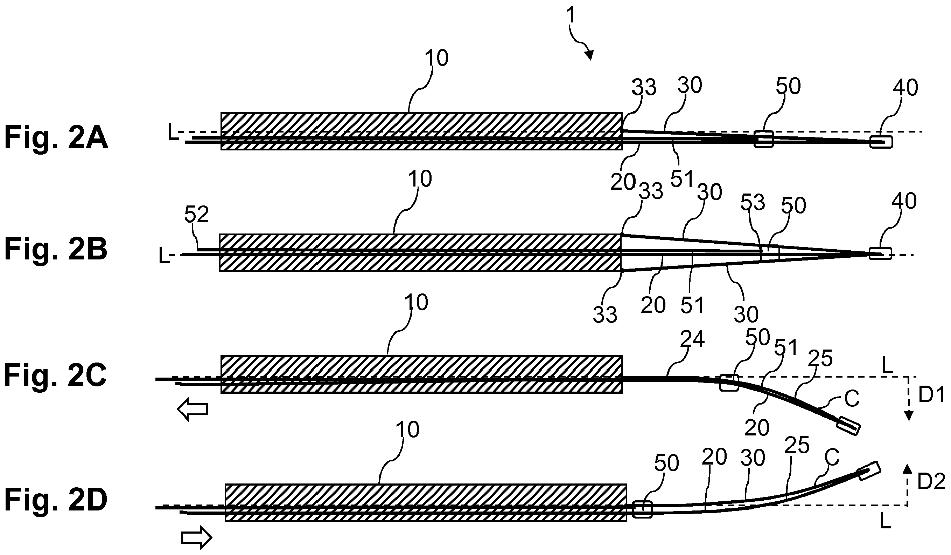

Fig. 1A-B and 2A-B display an internal armature 1 for a catheter 2 comprising a coil 10, a pull wire 20 and a connecting element 30 in side view.

The coil 10 extends along a longitudinal axis L from a proximal end 11 to a distal end 12. The term "proximal", as used herein, refers to the direction towards the physician when the internal armature 1 is used in a catheter 2 in catheterization of a patient, and the term "distal" refers to the opposite direction. The coil 10 further comprises an inner bore 13 extending along the longitudinal axis L. In other words, the coil 10 resembles a tube extending along the longitudinal axis L. The coil 10 consists of one or several elongated, threadlike members helically wound around the imaginary longitudinal axis L in a plurality of windings. Therein, the threadlike member may consist of a flat wire or any other suitable type of wire from a material including but not restricted to metals.

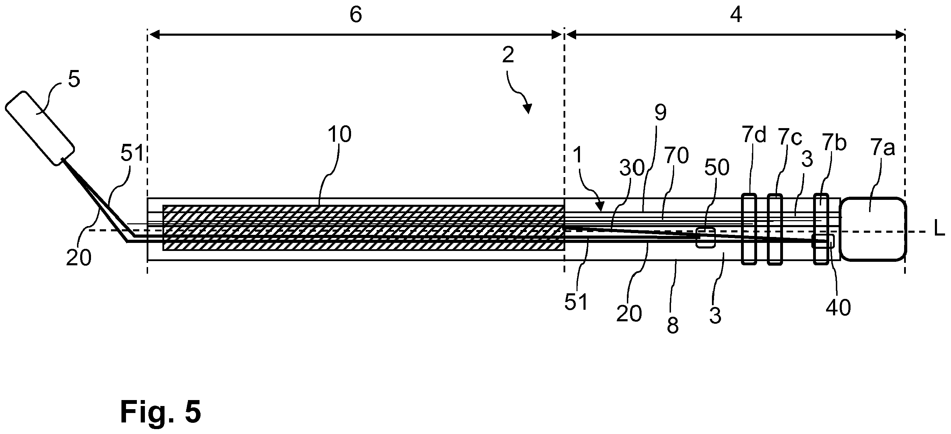

The internal armature 1 further comprises a pull wire 20 extending along the longitudinal axis L through the inner bore 13 of the coil 10. The pull wire 20 has a proximal end segment 21, a middle segment 22 and a distal end segment 23. The proximal end segment 21 can be connected to a manipulation device 5 as depicted in Fig. 5, such that the physician using a catheter 2 with the internal armature 1 may manipulate the pull wire 20. In Fig. 1 and 2, the proximal end segment 21 of the pull wire 20 is depicted shorter than in an actual catheter for better overview. In the case depicted in Fig. 1 and 2, the middle segment 22 of the pull wire 20 is positioned completely within the inner bore 13 of the coil 10. The distal end segment 23 of the pull wire 20 protrudes from the inner bore 13 at the distal end 12 of the coil 10 and further extends essentially parallel to the longitudinal axis L towards a sleeve 40, to which the distal tip 26 of the pull wire 20 is fixed i.e. by crimping, welding or soldering.

Fig. 1A-B and 2A-B further depict two connecting elements 30, each mechanically connecting the coil 10 to the pull wire 20. In respect of the connecting elements 30, Fig. 1A and 2A are side views and Fig. 1B and 2B are top views. In other words, the view of Fig. 1A or 2A, is rotated 90° around the longitudinal axis L compared to the view of Fig. 1B or 2B. Therefore, in Fig. 1A and 2A only one connecting element 30 is visible, since the connecting elements 30 are aligned on top of each other.

The connecting elements 30 each comprise a first end 31 and a second end 32 (see Fig. 1A). The respective first ends 31 are fixed to the coil 10 at connections 33, for example welding spots, and the second ends 32 of the connecting elements 30 are fixed, for example crimped, welded or soldered, to the sleeve 40. Thus, the second ends 32 of the connecting elements 30 are fixed to the pull wire 20 by means of the sleeve 40. Of course, the second ends 32 may also be directly connected to the pull wire 20, i.e. by welding, but the sleeve 40 facilitates fixing of the parts especially at small sizes.

The sleeve 40 may comprise an inner bore, into which the distal end 26 of the pull wire 20 and the second ends 32 of the connecting elements 30 can be positioned and subsequently crimped, welded or soldered to the sleeve 40.

In particular, the connecting elements 30 may be formed as braids or leaf springs. When a leaf spring is used as a connecting element 30 additional restoring forces are advantageously provided, such that the distal end segment 23 of the pull wire 20 returns to its straight confirmation in the absence of an external force.

Fig. 1C-D and Fig. 2 C-D illustrate the function of the internal armature 1 using the same side view perspective as in Fig. 1A and 2A. Arrows below the proximal end segment 21 of the pull wire 20 depict a movement of the pull wire 20 along the longitudinal axis L relative to the coil 10, which can be performed by a physician using a manipulation device 5 connected to the proximal end segment 21, as schematically depicted in Fig. 5. Accordingly, in Fig. 1C and 2C the pull wire 20 is pulled in the proximal direction, and in Fig. 1D and 2D the pull wire 20 is pushed in the distal direction.

As shown in Fig. 1C and 1D, the distal end segment 23 of the pull wire 20 is deflected along a curve C in a first lateral direction D1 if the pull wire 20 is pulled in the proximal direction (Fig. 1C) and deflected along a curve in a second lateral direction D2 if the pull wire 20 is pushed in the distal direction (Fig. 1D).

The first lateral direction D1 and the second lateral direction, D2 are each perpendicular to the longitudinal axis L and opposite to each other. In particular, the deflection of the pull wire 20 in opposite directions upon pushing or pulling may be mediated by a slightly asymmetric connection of the pull wire 20 to the connecting elements 30 (at the sleeve 40) or by different mechanical or geometric properties of the two connecting elements 30.

When the internal armature 1 is applied in a catheter 2 as depicted in Fig. 5 and explained below, deflection of the pull wire 20 results in deflection of the catheter tip section, particularly to adapt to curves and bends in the patient's vasculature or heart tissue.

Fig. 2 A-D depict a further embodiment of the internal armature 1 comprising an additional positioning element 50 and a positioning wire 51. Here, the positioning element 50 is formed as a positioning ring with an inner bore, through which the pull wire 20 extends, such that the positioning element 50 is slidably connected to the pull wire 20. In other words, the position along the longitudinal axis L of the positioning element 50 can be adjusted by sliding the positioning element 50 on the pull wire 20. To this end, a positioning wire 51 is provided, wherein the positioning wire 51 comprises a first end 52 to be connected to a manipulating device 5 (see Fig. 5) and a second end 53 which is fixed to the positioning element 50. During use of the catheter 2 (see Fig. 5), the physician may push or pull on the positioning wire 51 to adjust the axial position of the positioning element 50 on the pull wire 20. Fig. 2C shows a situation where the positioning element 50 is positioned approximately halfway between the distal end 12 of the coil 10 and the sleeve 40. The positioning element 50 divides the distal end segment 21 of the pull wire 20 into a first section 24 between the distal end 12 of the coil 10 and the positioning element 50 and a second section 25 between the positioning element 50 and the distal tip 26 of the pull wire 20 (at the sleeve 40).

Due to the positioning element 50, the first section 24 is kept in an essentially straight confirmation parallel to the longitudinal axis L, whereas the second section 25 is deflected along the curve C. In contrast, Fig. 2D shows a situation in which the positioning element 50 is arranged adjacent to the distal end 12 of the coil 10, and therefore almost the entire distal end segment 21 of the pull wire 20 consists of the second section 25. Accordingly, in Fig. 2D, almost the entire distal end segment 21 is deflected, resulting in a different shape of the curve C compared to Fig. 2C. In this manner, the positioning element 50 may be used to selectively adapt the curve shape of the catheter tip to the desired application. Alternatively, it is possible to fix the position of the positioning element 50 during assembly of the catheter. In this context, the internal armature 1 has the advantage that the same internal armature 1 may be used to build catheters for different applications by adjusting and fixing the positioning element 50 during manufacture.

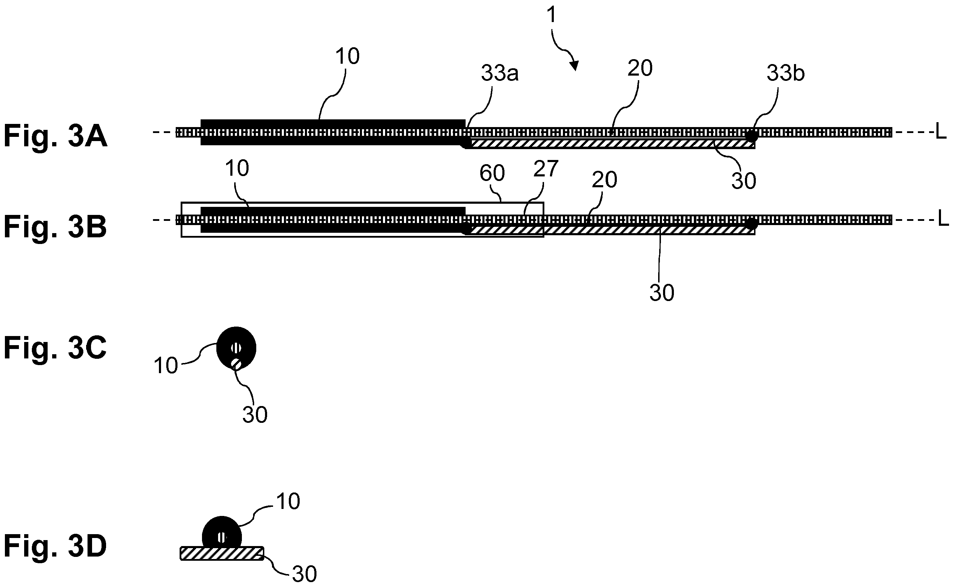

Fig. 3A-D depicts further embodiments of the internal armature 1 comprising a single connecting element 30. Fig. 3A is a side view of an embodiment of the internal armature 1 with a coil 10 having an inner bore 13 with a relatively small diameter, such that the pull wire 20 essentially completely fills the space of the inner bore 13. The single connecting element 30 is attached to the coil 10 and the pull wire 20 at connections 33, i.e. welding spots 33a, 33b.

The internal armature according to Fig. 3B comprises an additional bracing tube 60, i.e. from polyimide, placed over the coil 10 and a partial segment 27 of the pull wire 20. By means of the bracing tube 60, additional stiffening is provided, and the partial segment 27 is forced into a straight conformation, influencing the shape of the resulting curve C when the pull wire 20 is deflected.

Fig. 3C and 3D are top views viewed from the distal tip of the pull wire 20. In Fig. 3C the connecting element 30 is extended essentially parallel to the longitudinal axis L, similar to the situation depicted in Fig. 3A. In contrast, in Fig. 3D, the connecting element is positioned transversely to the longitudinal axis L. Note that this situation may occur in a catheter having a lumen with an inner diameter larger than the coil.

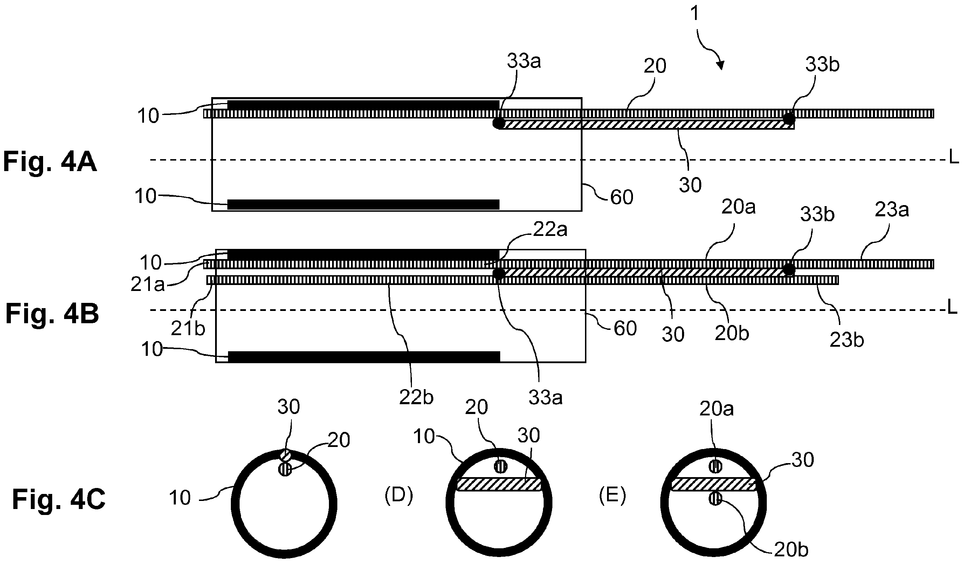

Fig. 4 shows further embodiments of the internal armature 1 with one connecting element 30, where the coil 10 has a larger diameter inner bore 13 compared to the embodiments depicted in Fig. 3, such that additional space is provided within the coil 10. For example, this space may provide an additional lumen 3 or several lumens 3 of a catheter 2 in which the internal armature 1 is used. In particular, the coil 10 may fill substantially the entire lumen of the exterior sheath or tube of the catheter, and additional tubes may be provided within the coil 10 to provide further lumens 3. Alternatively, the coil 10 may provide a lumen 3 within a larger exterior sheath or tube of the catheter 2.

Fig. 4A (sectional view), C and D (top views from the distal tip of the pull wire) show an embodiment with a single pull wire 20 as previously described. Fig. 4B (sectional view) and E (top view from the distal tip of the pull wire) depict an embodiment comprising a first pull wire 20a and a second pull wire 20b, each having a respective proximal end segment 21a, 21b, a respective middle segment 22a, 22b within the inner bore 13 (see Fig. 1) of the coil 10 and a respective distal end segment 23a, 23b protruding from the inner bore 13. In the embodiment depicted here, the connecting element 30 is sandwiched between the first pull wire 20a and the second pull wire 20b and both pull wires 20a, 20b are fixed to the connecting element 30 at two connections 33. Of course, other arrangements are also within the scope of the disclosure.

In particular, in the embodiment with a first pull wire 20a and a second pull wire 20b according to Fig. 4B, the first and second distal end segments 23a, 23b of the pull wires 20a, 20b may be jointly moved in a first lateral direction D1 perpendicular to the longitudinal axis L when the first pull wire 20a is moved, particularly in a proximal direction, relative to the coil 10, and the first and second distal end segments 23a, 23b of the pull wires 20a, 20b may be jointly moved in a second lateral direction D2 opposite the first lateral direction D1 when the second pull wire 20b is moved, particularly in a proximal direction, relative to the coil 10.

Both embodiments according to Fig. 4A and 4B comprise an additional bracing tube 60, i.e. from polyimide, placed over the coil 10, wherein a partial segment 27 of the pull wire 20 or partials segments 27 of the pull wires 20a, 20b in case of Fig. 4B is/are covered by the bracing tube 60. By means of the bracing tube 60, additional stiffening is provided, and the partial segment 27 is forced to a straight conformation, influencing the shape of the resulting curve C when the pull wire 20 is deflected or the pull wires 20a, 20b are deflected.

Fig. 5 shows a partially cut away side view of a catheter 2 comprising an internal armature 1, particularly as described above and shown in Fig. 1 to 4.

The catheter 2 comprises a relatively rigid shaft section 6, a deflectable tip section 4 distal to the shaft section 6 and a hollow external tube 8 defining a lumen 3. The coil 10, the pull wire 20 and the connecting element 30 of the internal armature 1 are positioned within the lumen 3 of the external tube 8. The coil 10 provides additional stiffness to the shaft section 6 of the catheter 2. As shown above in Fig. 2, the pull wire 20 and the connecting element 30 are connected and fixed by a sleeve 40 at the distal tip 26 of the pull wire 20. The internal armature 1 further comprises a positioning element 50 with an attached positioning wire 51 extending through the inner bore 13 of the coil parallel to the pull wire 20.

A manipulation device 5 is connected to the proximal end segment 21 (see Fig. 1 and 2) of the pull wire 20 and to the first end 52 (see Fig. 2) of the positioning wire 51, such that a physician operating the catheter 2 may control the pull wire 20 to deflect the tip section 4 of the catheter 2 and control the positioning wire 51 to adjust the shape of the curve C of the tip section 4.

The catheter 2 depicted in Fig. 5 is adapted for electrophysiological diagnosis and treatment of the heart and comprises a head electrode 7a as well as three ring electrodes 7b, 7c, 7d. Electrical connections leading to the electrodes 7a, 7b, 7c, 7d are placed in an internal tube 9 defining a respective smaller lumen 3 within the lumen 3 of the external tube 8. After the catheter 2 has been inserted into the vasculature of a patient and advanced to the heart, the electrodes 7a, 7b, 7c, 7d may be used to locate and ablate diseased heart tissue during electrophysiological treatment.

### List of reference numerals

- 1: Internal armature
- 2: Catheter
- 3: Lumen
- 4: Tip section
- 5: Manipulation device
- 6: Shaft section
- 7a, 7b, 7c, 7d: Electrode
- 8: External tube
- 9: Internal tube
- 10: Coil
- 11: Proximal end
- 12: Distal end
- 13: Inner bore
- 20, 20a, 20b: Pull wire
- 21, 21a, 21b: Proximal end segment
- 22, 22a, 22b: Middle segment
- 23, 23a, 23b: Distal end segment
- 24: First section
- 25: Second section
- 26: Distal tip
- 27: Partial segment
- 30: Connecting element
- 33a, 33b: Welding spots
- 31: First end of connecting element
- 32: Second end of connecting element
- 33: Connection
- 40: Sleeve
- 50: Positioning element
- 51: Positioning wire
- 52: First end of positioning wire
- 53: Second end of positioning wire
- 60: Bracing tube
- 70: Electrical connection
- C: Curve
- D1: First lateral direction
- D2: Second lateral direction
- L: Longitudinal axis

## Claims

1. An internal armature (1) for a catheter (2) comprising:
- a coil (10) configured to be positioned in a lumen (3) of a catheter (2), wherein the coil (10) extends along a longitudinal axis (L) from a proximal end (11) to a distal end (12), and wherein the coil (10) comprises an inner bore (13) extending along the longitudinal axis (L),
- at least one pull wire (20) for deflecting a tip section (4) of the catheter (2), wherein the pull wire (20) is at least partially positioned inside the inner bore (13) of the coil (10), and wherein the pull wire (20) comprises a proximal end segment (21) configured to be connected to a manipulation device (5) for moving the pull wire (20) and a distal end segment (23) protruding from the inner bore (13) at the distal end (12) of the coil (10),
wherein the internal armature (1) comprises at least one connecting element (30) connecting the coil (10) to the pull wire (20), wherein at least a part of the distal end segment (23) of the pull wire is deflectable along a curve (C) when the pull wire (20) is moved along the longitudinal axis (L) in respect of the coil (10).

2. The internal armature (1) according to claim 1, wherein the distal end segment (23) of the pull wire (20) is deflectable in a first lateral direction (D1) perpendicular to the longitudinal axis (L) when the pull wire (20) is moved along the longitudinal axis (L) towards the proximal end (11) of the coil (10), and wherein the distal end segment (23) of the pull wire (20) is deflectable in a second lateral direction (D2) perpendicular to the longitudinal axis (L) and opposite the first lateral direction (D1) when the pull wire (20) is moved along the longitudinal axis (L) towards the distal end (12) of the coil (10).

3. The internal armature (1) according to claim 1 or 2, wherein the internal armature (1) comprises a first pull wire (20a) and a second pull wire (20b), wherein the first pull wire (20a) and the second pull wire (20b) are each at least partially positioned inside the inner bore (13) of the coil (10), and wherein the first pull wire (20a) and the second pull wire (20b) are each connected to the coil (10) by means of the at least one connecting element (30), and wherein the first pull wire (20a) and the second pull wire (20b) each comprise a respective proximal end segment (21a, 21b) configured to be connected to a manipulation device (5) for moving the respective pull wire (20a, 20b) and a respective distal end segment (23a, 23b) protruding from the inner bore (13) at the distal end (12) of the coil (10), wherein the distal end segment (23a) of the first pull wire (20a) and the distal end segment (23b) of the second pull wire (20b) are jointly deflectable in a first lateral direction (D1) perpendicular to the longitudinal axis (L) when the first pull wire (20a) is moved along the longitudinal axis (L) in respect of the coil (10), and wherein the distal end segment (23a) of the first pull wire (20a) and the distal end segment (23b) of the second pull wire (20b) are jointly deflectable in a second lateral direction (D2) perpendicular to the longitudinal axis (L) and opposite the first lateral direction (D1) when the second pull wire (20b) is moved along the longitudinal axis (L) in respect of the coil (10).

4. The internal armature (1) according to any one of the preceding claims, wherein the connecting element (30) comprises a first end (31) and a second end (32), wherein the first end (31) is connected to the distal end (12) of the coil, and wherein the second end (32) is connected to the pull wire (20).

5. The internal armature (1) according to any one of the preceding claims, wherein the connecting element (30) is connected to the pull wire (20) by means of a sleeve (40), wherein particularly the connecting element (30) and/or the pull wire (20) is crimped, welded or soldered to the sleeve (40).

6. The internal armature (1) according to any one of the claims 1 to 5, wherein the connecting element (30) is a wire, particularly a metal wire, a wire strand or a braid.

7. The internal armature (1) according to any one of the claims 1 to 5, wherein the connecting element (30) is a spring element, particularly a leaf spring.

8. The internal armature (1) according to any one of the preceding claims, wherein the internal armature (1) comprises a positioning element (50), wherein a first section (24) of the pull wire (20) between the distal end (12) of the coil (10) and the positioning element (50) essentially extends along a straight line parallel to the longitudinal axis (L), and wherein a second section (25) of the pull wire (20) between the positioning element (50) and a distal tip (26) of the pull wire (20) is deflectable along a curve (C) when the pull wire (20) is moved along the longitudinal axis (L) in respect of the coil (10).

9. The internal armature (1) according to claim 8, wherein the positioning element (50) is slidably connected to the pull wire (20).

10. The internal armature (1) according to claim 8 or 9, wherein the internal armature (1) comprises a positioning wire (51) comprising a first end (52) configured to be connected to a manipulation device (5) for moving the positioning wire (51) and a second end (53) connected to the positioning element (50), such that the positioning element (50) is movable relative to the pull wire (20) by means of the positioning wire (51).

11. The internal armature (1) according to any one of the preceding claims, wherein the internal armature (1) comprises a bracing tube (60) positioned around a partial segment (27) of the pull wire (20) adjacent to the distal end (12) of the coil (10) in a circumferential direction in respect of the longitudinal axis (L), such that the partial segment (27) is positioned essentially along a straight line parallel to the longitudinal axis (L) by means of the bracing tube (60), wherein particularly the bracing tube (60) comprises polyimide or is formed from polyimide.

12. A catheter (2) comprising an internal armature (1) according to any one of the claims 1 to 11 and a lumen (3), wherein the coil (10) of the internal armature (1) is positioned in said lumen (3), and wherein the catheter (2) comprises a tip section (4) which is deflectable by deflecting at least a part of the distal end segment (23) of the pull wire (20) along a curve (C) when the pull wire (20) is moved along the longitudinal axis (L) in respect of the coil (10).

13. The catheter (2) according to claim 12, further comprising a manipulation device (5) connected to the proximal end segment (21) of the pull wire (20) of the internal armature, such that the pull wire (20) is movable along the longitudinal axis (L) by means of the manipulation device (5).

14. The catheter (2) according to claim 12 or 13, wherein the tip section (4) of the catheter (2) comprises at least one electrode (7a, 7b, 7c, 7d) for providing an electric current.

15. A method of manufacturing a catheter (2), wherein a single external tube (8) is provided, and wherein an internal armature (1) according to any one of the claims 1 to 11 is positioned within the external tube (8), wherein particularly the internal armature (1) is connected to the external tube (8).
